# FASCICULE DE BREVET EUROPEEN

(11) **EP 0 531 192 B1**
(45) Date de publication et mention de la délivrance du brevet: **13.09.1995**
(21) Numéro de dépôt: 92402361.7
(22) Date de dépôt: 28.08.1992
(51) Int. Cl.: A61K 7/00, A61K 7/42

(54) **Gel cosmétique solaire contenant en suspension des sphéroides hydratés d'une substance lipidique hydrophile et, en solution dans la phase aqueuse, un filtre U.V. de type sulfonique**
Kosmetische Lichtschutz Gele enthaltend suspendierte hydratierten Spheroide einer hydrophilen Lipidsubstanz und in Lösung der wässrigen Phase ein UV-Filter von dem Sulfonsäuretyp
Cosmetic sunblock gels containing in suspension hydrated spheroids of hydrophilic lipidic substance and in solution in this aqueous gel an UV-filter of the sulfonic type

(30) Priorité: 30.08.1991 FR 9110795
(43) Date de publication de la demande: 10.03.1993
(73) Titulaire: L'OREAL, 75008 Paris (FR)
(72) Inventeur: Ascione, Jean-Marc, F-75018 Paris (FR); Kauffmann, Myriam, F-69006 Lyon (FR)
(74) Mandataire: Stalla-Bourdillon, Bernard

(56) Documents cités:
- EP-A- 0 370 867
- EP-A- 0 412 865
- DE-A- 3 112 943
- US-A- 3 670 074

## Description

La présente invention a pour objet un gel cosmétique solaire contenant en suspension des sphéroïdes hydratés d'une substance lipidique hydrophile et, en solution dans la phase aqueuse, au moins un filtre U.V hydrosoluble du type sulfonique.

Le gel solaire selon l'invention est tout particulièrement destiné à protéger les matières kératiniques et plus particulièrement la peau et les cheveux des effets néfastes du rayonnement ultra-violet.

On rappellera que les radiations lumineuses de longueurs d'ondes comprises entre 280 nm et 400 nm permettent le brunissement de l'épiderme humain et que les rayons de longueurs d'ondes comprises entre 280 et 320 nm, connus sous la dénomination d'UV-B, provoquent des érythèmes et des brûlures cutanées qui peuvent nuire au développement du bronzage de sorte que ce rayonnement UV-B doit être filtré.

On sait par ailleurs que les rayons UV-A de longueurs d'ondes comprises entre 320 et 400 nm qui provoquent le brunissement de la peau, sont susceptibles d'induire une altération de celle-ci notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré de l'épiderme. Ils favorisent le déclenchement de la réaction érythémateuse ou amplifient cette réaction chez certains sujets et peuvent même être à l'origine de réactions phototoxiques ou photoallergiques. Il est donc tout à fait souhaitable de pouvoir filtrer le rayonnement UV-A.

De nombreux filtres U.V ont été proposés, ceux-ci étant soit liposolubles, soit hydrosolubles. Les filtres liposolubles présentent l'inconvénient de nécessiter des phases grasses qui confèrent à la peau et aux cheveux un aspect gras.

Les compositions solaires à bases de filtres U.V hydrosolubles sont de préférence des gels dont les propriétés sont particulièrement recherchées du fait de leur apport en eau conférant une sensation agréable de fraîcheur sur la peau et également du fait qu'elles ne confèrent pas un aspect gras à la peau ou aux cheveux.

Ces compositions aqueuses du type gel présentent toutefois certains inconvénients dans la mesure où elles peuvent provoquer un effet desséchant sur la peau conduisant à un certain manque de confort.

On vient maintenant de constater que l'on pouvait obtenir des compositions solaires sous forme de gel présentant d'excellentes propriétés cosmétiques et étant par ailleurs particulièrement stables dans le temps en utilisant une classe particulière de filtres U.V comportant une fonction acide sulfonique et par ailleurs, des sphéroïdes hydratés d'une substance lipidique hydrophile.

L'état de la technique relatif à des compositions solaires comportant en tant que filtres solaires des dérivés sulfonés est essentiellement constitué par EP-A-0.370.867.

Par ailleurs, il a déjà été proposé dans EP-A-0.412.865 d'utiliser dans des gels aqueux, en vue d'améliorer leurs propriétés, des sphéroïdes d'une substance lipidique non-hydrophile.

Les sphéroïdes hydratés de substances lipidiques hydrophiles du gel selon l'invention permettent d'empêcher l'effet de dessèchement de la peau et apportent une très agréable sensation de confort.

La présente invention a donc pour objet un gel cosmétique solaire contenant, en suspension dans la phase aqueuse continue, des sphéroïdes hydratés d'une substance lipidique hydrophile et en solution dans la phase aqueuse continue, au moins un filtre U.V à groupement acide sulfonique choisi dans le groupe constitué par l'acide 2-hydroxy 4-méthoxybenzophénone 5-sulfonique, l'acide 2-phényl benzimidazole 5-sulfonique et l'acide benzène 1,4- di(3-méthylidène camphosulfonique) et au moins un agent gélifiant.

Les études qui ont été réalisées ont en effet permis de mettre en évidence que la stabilité des gels selon l'invention dépendait du choix des filtres U.V et qu'une bonne stabilité ne pouvait être obtenue qu'avec une sélection très limitée de certains filtres U.V appartenant tous d'ailleurs à la classe des composés à fonction acide sulfonique.

Les sphéroïdes hydratés d'une substance lipidique hydrophile du gel selon l'invention sont comparables à de petits réservoirs ou globules, stériquement bien délimités d'un diamètre moyen de particules compris entre 50 et 10.000 µm et de préférence entre 100 et 5.000 µm.

La phase aqueuse du gel ou phase continue peut être de l'eau ou un mélange d'eau et d'un solvant organique hydroxylé tel que l'alcool éthylique, la glycérine, le sorbitol, les glycols tel que le propylène glycol ou les éthers de glycol tel que le monoéthyléther de diéthylène glycol. De façon préférentielle, la phase aqueuse contient au moins 70 % en poids d'eau.

Parmi les agents gélifiants utilisables selon l'invention, on peut citer les polymères carboxyvinyliques tels que les carbopols, les biopolysaccharides tels que les gommes de xanthane et les scléroglucanes, les dérivés de cellulose, les gélifiants minéraux, une dispersion aqueuse de copolymère d'acrylate d'ammonium/acrylamide réticulé par un agent de réticulation à polyinsaturation oléfinique, dispersé dans une émulsion eau-dans-l'huile tel que le produit "PAS 5161" commercialisé par la Société HOECHST ou un gel résultant de l'interaction ionique d'un polymère cationique constitué par un copolymère de cellulose ou d'un dérivé de cellulose greffé avec un sel de monomère hydrosoluble d'ammonium quaternaire et d'un polymère anionique carboxylique ayant une viscosité capillaire absolue dans le diméthylformamide ou le méthanol, à une concentration de 5 % et à 30°C, inférieure ou égale à 30 x 10⁻³P.a.s..

La concentration du gel en agent gélifiant est généralement comprise entre 0,1 et 20 % et de préférence entre 0,1 et 10 %.

Les filtres U.V. du gel selon l'invention tels que mentionnés ci-dessus sont disponibles auprès des fournisseurs suivants :

L'acide 2-hydroxy 4-méthoxybenzophénone 5-sulfonique est vendu sous la dénomination de "UVINUL MS 40" par la Société BASF,

l'acide 2-phényl benzimidazole 5-sulfonique est vendu sous la dénomination de "EUSOLEX 232" par la Société MERCK, et

l'acide benzène 1,4[di(3-méthylidène camphosulfonique)], partiellement ou totalement neutralisé, a été décrit dans le brevet français n° 2.528.420.

Les filtres U.V du gel selon l'invention peuvent être présents en une proportion comprise entre 0,1 et 10 % et de préférence entre 0,5 et 5 % en poids par rapport au poids total du gel.

Par l'expression "sphéroïdes" telle qu'utilisée selon l'invention, on doit entendre de petites particules solides essentiellement sphériques alors que par l'expression "sphéroïdes hydratés" on doit entendre de petites particules crémeuses également essentiellement sphériques contenant de l'eau.

Le diamètre moyen des sphéroïdes que l'on incorpore dans la composition peut être compris dans de très larges limites, mais celui-ci doit être tel qu'après hydratation, les sphéroïdes hydratés aient un diamètre moyen des particules compris dans la gamme indiquée ci-dessus et de préférence entre 100 et 5 000 µm.

En fonction de la nature de la substance lipidique hydrophile utilisée, le gonflement des sphéroïdes par hydratation a pour effet d'augmenter voire de doubler le diamètre moyen des sphéroïdes solides introduits dans la composition.

En effet, la capacité d'hydratation des sphéroïdes est telle qu'ils peuvent absorber 1,3 à 8 fois leur poids d'eau, c'est-à-dire que leur volume est multiplié par un facteur compris entre 1,3 et 8 si l'on néglige les différences de densité. Par conséquent, leur diamètre est multiplié par la racine cubique de ce facteur, soit 1,1 à 2.

D'autre part, la nature de la phase externe aqueuse et la température peuvent moduler l'absorption d'eau par les sphéroïdes.

Le pourcentage en poids des sphéroïdes introduits dans le gel à l'état solide est bien entendu fonction de l'effet recherché, un pourcentage élevé conférant un effet adoucissant et lubrifiant plus prononcé.

Dans la pratique, ce pourcentage en poids est généralement compris entre 0,1 et 50 % mais de préférence entre 1 et 10 % en poids.

Les substances lipidiques hydrophiles pour la formation des sphéroïdes doivent être solides à température ambiante, c'est-à-dire présenter un point de fusion supérieur à 20 °C. Le point de fusion maximum n'est pas critique mais l'on préfère utiliser des substances lipidiques hydrophiles ayant un point de fusion inférieur à 80 °C.

Parmi les substances lipidiques solides hydrophiles particulièrement préférées pour la préparation des sphéroïdes, on peut notamment mentionner :
(1) Les alcools gras en C₁₂-C₂₄ ayant un point de fusion compris entre 20 et 80 °C et ayant un indice d'hydroxyle (IOH) compris entre 100 et 300.
   Parmi ces alcools gras ceux particulièrement préférés sont : l'alcool myristique, l'alcool cétylique et l'alcool stéarylique.
(2) Les esters partiels d'acides gras en C₁₂-C₂₄ avec des polyols ou oligomères de polyols tels que l'éthylène glycol, le propylène glycol, le glycérol, des sucres en C₃-C₆ linéaires, ramifiés ou cycliques ou le diéthylène glycol, les polyéthylène glycols, les polyglycérols et le saccharose.
   Ces esters partiels doivent avoir un point de fusion compris entre 20 et 80 °C et un indice d'hydroxyle (IOH) compris entre 50 et 500 cu une HLB (Hydrophile-Lipophile Balance) comprise entre 1 et 13.
   Parmi ces esters partiels on peut notamment citer : le monodipalmitostéarate de glycérol tel que le "GELEOL" vendu par la Société GATTEFOSSE, le monopalmitate de sorbitan tel que l' "ARLACEL 40" vendu par la Société ICI, le monostéarate de diéthylène glycol tel que le "TEIGIN D" vendu par la Société GOLDSCHMIDT ou le béhénate de glycérol tel que le "COMPRITOL" vendu par la Société GATTEFOSSE.
(3) Les dérivés oxyéthylénés de corps gras tels que ceux d'alcools gras en C₁₂-C₂₄ et de leurs esters, d'acides gras en C₁₂-C₂₄ et de leurs esters, d'amines et amides gras en C₁₂-C₂₄, de cires, de lanoline et d'huiles hydrogénées et leurs mélanges, ces corps gras étant oxyéthylénés à l'aide de 2 à 50 moles d'oxyde d'éthylène (OE) par mole de corps gras.
   Ces dérivés oxyéthylénés de corps gras doivent avoir un point de fusion de 20 à 80 °C et une HLB comprise entre 1 et 13.
   Parmi ceux-ci on peut notamment mentionner : l'alcool stéarylique oxyéthyléné à 2 moles d'OE, tel que le "BRIJ 72" vendu par la Société ICI, le distéarate de polyéthylèneglycol à 8 moles d'OE, tel que le "LIPOPEG 4 DS" vendu par la Société LIPO ou la cire d'abeilles hydrophile telle que l' "APIFIL" vendue par la Société GATTEFOSSE.
(4) Les produits issus de la réaction d'alcoolyse entre les triglycérides et les polyéthylèneglycols, ayant un point de fusion compris entre 20 et 80 °C, et un indice d'hydroxyle compris entre 50 et 500 ou une HLB comprise entre 1 et 13.
   Il s'agit en particulier des glycérides lauropalmitostéariques polyoxyéthylénés glycolysés (huiles de palme/palmiste hydrogénées interestérifiées), tels que les "LABRAFILS M2130 BS, M2130 CS et WL2514 CS", vendus par la Société GATTEFOSSE ou des glycérides hydroxystéariques polyoxyéthylénés glycolysés (huile de ricin hydrogénée interestérifiée) tel que le "LABRAFIL WL 1958 CS" vendu par la Société GATTEFOSSE.
(5) Les phospholipides et sphingolipides et leurs dérivés hydrogénés de point de fusion supérieur à 20 °C, ayant un indice d'hydroxyle compris entre 50 et 500 ou une HLB comprise entre 1 et 13.
(6) Les silicones amphiphiles tels que les diméthicone copolyols, les polyalkyl diméthicone copolyols, ou leurs dérivés portant des esters à longue chaîne, comportant 2 à 50 moles d'OE par mole de produit, et ayant un point de fusion compris entre 20 et 80 °C ou une stéaroxydiméthicone.

Les substances lipidiques hydrophiles telles qu'énumérées ci-dessus peuvent être utilisées seules ou éventuellement sous forme d'un mélange.

Il est également possible d'utiliser des mélanges de substances lipidiques, chacune d'elles pouvant avoir une ou plusieurs caractéristiques (point de fusion, indice d'hydroxyle, HLB) hors des limites précédemment définies, mais dont l'association possède les caractéristiques physico-chimiques énoncées.

Il est en outre possible pour ajuster la consistance ou la viscosité des sphéroïdes, d'introduire dans le mélange, des argiles modifiés ou leur dispersion huileuse, des silices, des savons métalliques ou tout autre structurant des corps gras.

Les sphéroïdes hydratés de substance lipidique hydrophile peuvent également contenir dissous ou dispersés dans leur matrice différents adjuvants cosmétiquement acceptables, liposolubles ou non liposolubles, tels que des vitamines ou pro-vitamines en particulier les vitamines A2 et E et leurs esters, les esters de la vitamine C, des caroténoïdes, des substances anti-radicalaires, des filtres U.V liposolubles, des molécules agissant sur la pigmentation, sur l'inflammation, des esters gras émollients, des huiles d'origine minérale, animale, végétale ou synthétique, des céramides, des extraits biologiques, des colorants du cheveu, des pigments, des antioxydants, des conservateurs.

Il va de soi que lorsque l'on incorpore ou charge de tels composants cosmétiques dans les sphéroïdes de substance lipidique hydrophile, il convient de choisir une substance lipidique hydrophile appropriée de telle sorte qu'après granulation, les sphéroïdes se présentent toujours sous forme solide avant leur incorporation dans la composition, et conservent toujours leur capacité d'absorption d'eau.

La phase aqueuse du gel peut également contenir divers adjuvants cosmétiquement acceptables hydrosolubles, tels que des agents hydratants, des conservateurs, des agents acidifiants ou alcalinisants, des polymères, des colorants, des vitamines, des antiradicaux libres, des anti-inflammatoires, des extraits biologiques à condition qu'ils ne destabilisent pas la composition selon l'invention.

Le procédé de préparation des gels tels que définis ci-dessus consiste à incorporer, sous agitation, les sphéroïdes solides, chargés ou non chargés, dans la phase continue gélifiée renfermant le filtre à groupement(s) sulfonique(s) de façon à former une suspension et à la soumettre ensuite à une étape dite de "maturation" pendant une durée déterminée et à une température contrôlée, le traitement étant fonction de la nature de la substance lipidique hydrophile utilisée pour former les sphéroïdes et de la nature de la phase aqueuse externe.

Cette dernière étape dite de "maturation" provoque l'hydratation des sphéroïdes et a pour conséquence leur gonflement et leur ramollissement.

Le phénomène qui est visible macroscopiquement, s'accompagne d'une augmentation du diamètre et d'une modification éventuelle de la couleur et de l'opacité des sphéroïdes notamment lorsqu'ils sont chargés en une substance colorante ou en pigment(s).

L'étape de maturation est généralement effectuée à une température comprise entre 15 et 80 °C et pendant une durée de 1 heure à 15 jours.

Les sphéroïdes solides peuvent être préparés par toutes méthodes conventionnelles de granulation ou de sphéronisation à chaud ou à froid.

Par exemple, par solidification par le froid de gouttelettes de la substance lipidique hydrophile fondue, maintenue en mouvement dans un liquide non solvant tel que l'eau, ou par refroidissement par un gaz froid de gouttelettes de la substance lipidique hydrophile pulvérisée, projetée sur un disque rotatif, ou extrudée, ou par granulation à chaud autour d'un noyau solide dans une turbine ou par un lit d'air fluidisé, ou dans un mélangeur planétaire, par passage à travers une filière ou une grille vibrante, par moulage, coulage ou injection à chaud ou sous pression dans des moules, ou par découpage ou division d'une masse lipidique solide.

Les méthodes de préparation à froid, ou requérant peu d'échauffement, sont généralement préférées lorsque les composants cosmétiques ou dermo-pharmaceutiques inclus dans la substance lipidique hydrophile sont fragiles vis-à-vis de la température ou de l'oxydation. Le refroidissement des gouttelettes fondues est obtenu de préférence par un gaz plutôt que par l'eau.

Selon le procédé choisi et les paramètres de fabrication, les sphéroïdes solides obtenus sont parfaitement calibrés ou de distribution de taille ou de diamètre plus ou moins étalée.

Les sphéroïdes solides peuvent être manipulés industriellement sans précaution particulière. Leur introduction dans la phase externe peut s'effectuer directement dans le conditionnement définitif, dans lequel l'étape de maturation aura lieu.

L'invention a également pour objet un procédé de protection des matières kératiniques, telles que la peau et les cheveux vis-à-vis du rayonnement ultra-violet, consistant à appliquer sur celles-ci une quantité efficace de gel tel que défini ci-dessus.

### PROCEDE DE PREPARATION DES SPHEROÏDES SOLIDES

L'ensemble substance(s) lipidique(s) hydrophile(s) + adjuvant(s) cosmétique(s) est préparé à chaud. Le ou les composants solides de la substance lipidique hydrophile sont fondus à une température supérieure de 2 à 3 °C à celle de la substance lipidique hydrophile du plus haut point de fusion. Puis on ajoute les autres composants, en commençant par les moins fragiles.

Le mélange est maintenu à une température supérieure de 2 à 3 °C à celle de la zone de solidification. Les composants fragiles ou volatils sont ajoutés en dernier. Les solides sont ajoutés à la fin dans le mélange fondu, et maintenus sous agitation. Ils peuvent être éventuellement "empatés" par une fraction de la substance lipidique hydrophile fondue avant leur incorporation à la totalité du mélange.

Lorsque le mélange est homogène, la masse fondue est pulvérisée dans la partie supérieure d'une colonne verticale d'air froid. Les gouttelettes de lipides fondus tombent par gravitation tout en refroidissant.

Les gouttelettes solidifiées sont récupérées à la base de la colonne.

Selon un autre procédé, il est également possible de couler la masse lipidique fondue dans une phase aqueuse portée à la même température et maintenue sous agitation circulaire : le mélange lipidique se disperse sous forme de gouttelettes sphériques. Un agent gélifiant est alors ajouté dans le milieu, qui est refroidi sous agitation. Dans ce cas, la préparation des sphéroïdes est réalisée simultanément à leur incorporation dans le milieu externe final.

On va maintenant donner à titre d'illustration et sans aucun caractère limitatif, plusieurs exemples de gels solaires selon l'invention.

### EXEMPLE 1 :

On prépare un gel solaire pour la protection de la peau de la façon suivante :
Dans un flacon approprié, on conditionne 98,5 g du gel ayant la composition suivante :

| | |
|---|---|
| - Acide benzène 1,4-[di(3-méthylidène camphosulfonique)] | 5 g MA |
| - Acide polyacrylique réticulé (PM 4000000) vendu sous la dénomination "CARBOPOL 940" par la Société GOODRICH | 1 g |
| - Sorbitol en solution aqueuse à 70 % MA | 3,5 g MA |
| - Conservateur qs | |
| - Triéthanolamine qs pH = 7 | |
| - Eau qsp | 100 g |

A ce gel, on incorpore sous agitation 1,5 g de sphéroïdes solides ayant la composition suivante :

| | |
|---|---|
| - Mono et distéarate de glycérol vendu sous la dénomination "GELEOL" par la Société GATTEFOSSE | 58,3 g |
| - Mono et dipalmitostéarate de polyéthylèneglycol (6-32 moles d'oxyde d'éthylène) vendu sous la dénomination "TEFOSE 1500" par la Société GATTEFOSSE | 15,3 g |
| - Mélange de stéaroxydiméthicone et d'alcool stéarylique vendu sous la dénomination "BELSIL SDM 6022" par la Société WACKER | 15,3 g |
| - p-méthoxycinnamate de 2-éthylhexyle vendu sous la dénomination "PARSOL MCX" par la Société GIVAUDAN | 8 g |
| - 2-hydroxy 4-méthoxybenzophénone | 2 g |
| - Parfum qs | |
| - Colorant qs | |

Le flacon ainsi conditionné est alors soumis à l'étape de maturation pendant 48 h à température ambiante.

Cette composition est stable pendant au moins 2 mois à 45°C.

### EXEMPLE 2 :

On prépare un gel solaire pour la protection de la peau de la façon suivante :
Dans un flacon approprié, on conditionne 98,5 g du gel ayant la composition suivante :

| | |
|---|---|
| - Acide 2-phényl benzimidazole 5-sulfonique vendu sous la dénomination "EUSOLEX 232" par la Société MERCK | 5 g MA |
| - Acide polyacrylique réticulé (PM 4000000) vendu sous la dénomination "CARBOPOL 940" par la Société GOODRICH | 1 g |
| - Sorbitol en solution aqueuse à 70 % MA | 3,5 g MA |
| - Conservateur qs | |
| - Triéthanolamine qs pH = 7 | |
| - Eau qsp | 100 g |

A ce gel, on incorpore sous agitation 1,5 g des sphéroïdes solides décrits à l'exemple 1.

Le flacon ainsi conditionné est alors soumis à l'étape de maturation pendant 48 h à température ambiante.

Cette composition est stable pendant au moins 2 mois à 45°C.

### EXEMPLE 3 :

On prépare un gel solaire pour la protection des cheveux de la façon suivante :
Dans un flacon approprié, on conditionne 98,5 g du gel ayant la composition suivante :

| | |
|---|---|
| - Acide 2-hydroxy 4-méthoxybenzophénone 5-sulfonique vendu sous la dénomination "UVINUL MS 40" par la Société BASF | 2 g MA |
| - Acide polyacrylique réticulé (PM 4000000) vendu sous la dénomination "CARBOPOL 940" par la Société GOODRICH | 1 g |
| - Sorbitol en solution aqueuse à 70 % MA | 3,5 g MA |
| - Conservateurs qs | |
| - Triéthanolamine qs pH = 7 | |
| - Eau qsp | 100 g |

A ce gel, on incorpore sous agitation 1,5 g de sphéroïdes solides décrits à l'exemple 1.

Le flacon ainsi conditionné est alors soumis à l'étape de maturation pendant 48 h à température ambiante.

Cette composition est stable pendant au moins 2 mois à 45°C.

### EXEMPLE 4 :

On prépare un gel solaire pour la protection de la peau de la façon suivante :
Dans un flacon approprié, on conditionne 98,5 g de gel ayant la composition suivante :

| | |
|---|---|
| - Acide benzène 1,4-[di(3-méthylidène camphosulfonique)] | 5 g MA |
| - Acide polyacrylique réticulé (PM 4000000) vendu sous la dénomination "CARBOPOL 940" par la Société GOODRICH | 1 g |
| - Sorbitol en solution aqueuse à 70 % | 3,5 g MA |
| - Conservateur qs | |
| - Triéthanolamine qs pH = 7 | |
| - Eau qsp | 100 g |

A ce gel, on incorpore sous agitation 1,5 g de sphéroïdes solides ayant la composition suivante :

| | |
|---|---|
| - Mono et distéarate de glycérol vendu sous la dénomination "GELEOL" par la Société GATTEFOSSE | 71,5 g |
| - Huile de ricin hydrogénée interestérifiée vendue sous la dénomination "LABRAFIL WL 1958 CS" par la Société GATTEFOSSE | 17,5 g |
| - p-méthoxycinnamate de 2-éthylhexyle vendu sous la dénomination "PARSOL MCX" par la Société GIVAUDAN | 8 g |
| - 2-hydroxy 4-méthoxybenzophénone | 2 g |
| - Parfum qs | |
| - Colorant qs | |

L'étape de maturation a lieu dans une enceinte appropriée à une température de 45°C pendant 48 h.

Cette composition est stable pendant au moins 2 mois à 45°C.

## Revendications

1. Gel cosmétique solaire caractérisé par le fait qu'il contient en suspension dans la phase aqueuse continue des sphéroïdes hydratés d'une substance lipidique hydrophile et en solution dans la phase aqueuse continue, au moins un filtre U.V. à groupement acide sulfonique choisi dans le groupe constitué par l'acide 2-hydroxy 4-méthoxy benzophénone 5-sulfonique, l'acide 2-phényl benzimidazole 5-sulfonique et l'acide benzène 1,4-[di(3-méthylidène camphosulfonique)] et au moins un agent gélifiant.

2. Gel selon la revendication 1, caractérisé par le fait que la phase aqueuse continue est de l'eau ou un mélange d'eau et d'un solvant organique hydroxylé, ledit mélange contenant au moins 70 % en poids d'eau.

3. Gel selon la revendication 1, caractérisé par le fait que les sphéroïdes hydratés ont un diamètre moyen des particules compris entre 50 et 10.000 µm et de préférence entre 100 et 5.000 µm.

4. Gel selon l'une quelconque des revendications précédentes, caractérisé par le fait que la substance lipidique hydrophile a un point de fusion compris entre 20°C et 80°C.

5. Gel selon l'une quelconque des revendications précédentes, caractérisé par le fait que l'agent gélifiant est présent à une concentration comprise entre 0,1 et 20 % et de préférence entre 0,1 et 10 % en poids par rapport au poids total du gel.

6. Gel selon l'une quelconque des revendications précédentes, caractérisé par le fait que le filtre U.V. est présent en une proportion comprise entre 0,1 et 10 % et de préférence entre 0,5 et 5 % en poids par rapport au poids total du gel.

7. Gel selon l'une quelconque des revendications précédentes, caractérisé par le fait que la concentration des sphéroïdes est comprise entre 0,1 et 50 % et de préférence entre 1 et 10 % en poids par rapport au poids total du gel.

8. Gel selon l'une quelconque des revendications précédentes, caractérisé par le fait que les sphéroïdes hydratés sont chargés en au moins un adjuvant cosmétique liposoluble ou non liposoluble.

9. Gel selon l'une quelconque des revendications précédentes, caractérisé par le fait que la phase aqueuse continue contient en outre au moins un adjuvant cosmétique hydrosoluble.

10. Utilisation d'un gel tel que défini selon l'une quelconque des revendications 1 à 9, pour la preparation d'une composition pour la protection des cheveux et de la peau vis-à-vis du rayonnement ultra-violet.

## Claims

1. Antisun cosmetic gel, characterized in that it contains, suspended in the continuous aqueous phase, hydrated spheroids of a hydrophilic lipid substance and, in solution in the continuous aqueous phase, at least one UV screening agent containing a sulphonic acid group chosen from the group consisting of 2-hydroxy-4-methoxybenzophenone-5-sulphonic acid, 2-phenylbenzimidazole-5-sulphonic acid and benzene-1,4-[di(3-methylidenecamphorsulphonic)] acid and at least one gelling agent.

2. Gel according to Claim 1, characterized in that the continuous aqueous phase is water or a mixture of water and a hydroxylated organic solvent, the said mixture containing at least 70 % by weight of water.

3. Gel according to Claim 1, characterized in that the hydrated spheroids have a mean particle diameter of between 50 and 10,000 µm, and preferably of between 100 and 5,000 µm.

4. Gel according to any one of the preceding claims, characterized in that the hydrophilic lipid substance has a melting point of between 20°C and 80°C.

5. Gel according to any one of the preceding claims, characterized in that the gelling agent is present in a concentration between 0.1 and 20 %, and preferably between 0.1 and 10 %, by weight relative to the total weight of the gel.

6. Gel according to any one of the preceding claims, characterized in that the UV screening agent is present in a proportion between 0.1 and 10 %, preferably between 0.5 and 5 %, by weight relative to the total weight of the gel.

7. Gel according to any one of the preceding claims, characterized in that the concentration of the spheroids is between 0.1 and 50 %, and preferably between 1 and 10 %, by weight relative to the total weight of the gel.

8. Gel according to any one of the preceding claims, characterized in that the hydrated spheroids are charged with at least one lipid-soluble or lipid-insoluble cosmetic adjuvant.

9. Gel according to any one of the preceding claims, characterized in that the continuous aqueous phase additionally contains at least one water-soluble cosmetic adjuvant.

10. Use of a gel as defined according to any one of Claims 1 to 9, for the preparation of a composition for the protection of the hair and the skin against ultraviolet radiation.

## Patentansprüche

1. Kosmetisches Lichtschutzgel, dadurch gekennzeichnet, daß es suspendiert in einer kontinuierlichen wäßrigen Phase hydratisierte Kügelchen einer hydrophilen Lipidsubstanz und gelöst in der kontinuierlichen wäßrigen Phase mindestens einen UV-Filter aus der Gruppe der Sulfonsäuren, der aus der aus 2-Hydroxy-4-methoxybenzophenon-5-sulfonsäure, 2-Phenylbenzimidazol-5-sulfonsäure und Benzol-(di[3-methylidenkamplersulfonsäure]) bestehenden Gruppe ausgewählt ist, und mindestens ein Geliermittel enthält.

2. Gel gemäß Anspruch 1, dadurch gekennzeichnet, daß die kontinuierliche wäßrige Phase aus Wasser oder einem Gemisch aus Wasser und einem organischen hydroxylierten Lösungsmittel besteht, wobei das Gemisch mindestens 70 Gew.-% Wasser enthält.

3. Gel gemäß Anspruch 1, dadurch gekennzeichnet, daß die hydratisierten Kügelchen einen mittleren Teilchendurchmesser zwischen 50 und 10000 µm, vorzugsweise zwischen 100 und 5000 µm aufweisen.

4. Gel gemäß einem der vorstehenden Ansprüche, dadurch gekennzeichnet, daß die hydrophile Lipidsubstanz einen Schmelzpunkt zwischen 20 und 80 °C aufweist.

5. Gel gemäß einem der vorstehenden Ansprüche, dadurch gekennzeichnet, daß das Geliermittel in einer Konzentration von 0,1 bis 20 %, vorzugsweise 0,1 bis 10 % vorliegt, bezogen auf das Gesamtgewicht des Gels.

6. Gel gemäß einem der vorstehenden Ansprüche, dadurch gekennzeichnet, daß der UV-Filter in einer Menge von 0,1 bis 10 % und vorzugsweise von 0,5 bis 5 %, bezogen auf das Gesamtgewicht des Gels, vorliegt.

7. Gel gemäß einem der vorstehenden Ansprüche, dadurch gekennzeichnet, daß die Konzentration an Kügelchen zwischen 0,1 und 50 % und vorzugsweise zwischen 1 und 10 %, bezogen auf das Gesamtgewicht des Gels, liegt.

8. Gel gemäß einem der vorstehenden Ansprüche, dadurch gekennzeichnet, daß die hydratisierten Kügelchen mit mindestens einem fettlöslichen oder fettunlöslichen kosmetischen Hilfsstoff beladen sind.

9. Gel gemäß einem der vorstehenden Ansprüche, dadurch gekennzeichnet, daß die kontinuierliche wäßrige Phase zusätzlich mindestens einen wasserlöslichen kosmetischen Hilfsstoff enthält.

10. Verwendung eines Gels gemäß einem der Ansprüche 1 bis 9 zur Herstellung einer Zubereitung zum Schutz der Haare und der Haut gegen UV-Strahlen.
